Europäisches Patentamt

European Patent Office

Office européen des brevets

(19)

(11) EP 0 554 910 B1

(12) **EUROPÄISCHE PATENTSCHRIFT**

(45) Veröffentlichungstag und Bekanntmachung des Hinweises auf die Patenterteilung:
**10.09.1997 Patentblatt 1997/37**

(51) Int Cl.6: **C12Q 1/58**, B08B 9/46, G01N 21/90

(21) Anmeldenummer: **93101867.5**

(22) Anmeldetag: **06.02.1993**

(54) **Verfahren und Vorrichtung zur Detektion von Urin**

Method and device for detecting urine

Méthode et dispositif pour la détection d'urine

(84) Benannte Vertragsstaaten:
**AT BE CH DE DK ES FR GB GR IE IT LI NL PT SE**

(30) Priorität: **06.02.1992 DE 4203274**

(43) Veröffentlichungstag der Anmeldung:
**11.08.1993 Patentblatt 1993/32**

(73) Patentinhaber: **Krieg, Gunther, Prof.Dr.Ing.
D-76227 Karlsruhe (DE)**

(72) Erfinder:
  • **Krieg, Gunther, Prof. Dr.Ing.
    W-7500 Karlsruhe 41 (DE)**
  • **Koukolitschek, Karl, Dipl.-Ing.(FH)
    W-7500 Karlsruhe 21 (DE)**
  • **Maier, Wilfried
    W-7519 Sulzfeld (DE)**

(74) Vertreter: **Dipl.-Ing. Heiner Lichti
Dipl.-Phys. Dr.rer.nat. Jost Lempert Dipl.-Ing.
Hartmut Lasch
Postfach 41 07 60
76207 Karlsruhe (DE)**

(56) Entgegenhaltungen:
EP-A- 0 306 307          WO-A-88/00862
GB-A- 2 196 117          US-A- 3 417 241

  • **JOURNAL OF THE ASSOCIATION OF OFFICIAL ANALYTICAL CHEMISTS Bd. 63, Nr. 5, 1980 (US), S. 985-987, R.K.S. GOO et al.**
  • **PATENT ABSTRACTS OF JAPAN vol. 8, no. 223 (P-307)12. Oktober 1984**
  • **PATENT ABSTRACTS OF JAPAN vol. 10, no. 111 (P-451)25. April 1986**

**Beschreibung**

Die Erfindung betrifft ein Verfahren und eine Vorrichtung zur Detektion und zur quantitativen Analyse von Resten von Urin in Behältern, wie Flaschen, insbesondere in der Getränkeindustrie.

Mit der zunehmenden Verbreitung von Kunststoffflaschen und Kunststoffbehältern in der Lebensmittel- und Getränkeindustrie, speziell auch in Getränke-Abfüllinien, z.B. unter Einsatz von Polyethylen und anderen Kunststoffen, wächst das Risiko der Kontamination von Flaschen und Behältern mit Schadstoffen bzw. Fremdgerüchen. Gemäß neuen Erfahrungen spielen neben chemischen Schadstoffen insbesondere Kontaminationen durch Urin eine ganz wesentliche Rolle.

Während viele Schadstoffe relativ hohe Partialdrükke aufweisen und daher in der Gasphase direkt nachgewiesen werden können, ist dies bei Urin mit großen Schwierigkeiten verknüpft, da im allgemeinen keine urinspezifischen Stoffe mit genügend hoher Konzentration in der Gasphase vorliegen. Letzteres ist insbesondere dann der Fall, wenn, z.B. durch Trocknungsprozesse bedingt, keine Restflüssigkeit mehr vorliegt. Die Schwierigkeiten bei der Urin-Gasphasendetektion liegen unter anderem auch darin, daß:

- der Hauptbestandteil von Urin, d.h. Harnstoff, bei Zimmertemperatur einen verschwindend kleinen Dampfdruck aufweist,

- an sich sehr leicht nachweisbare Urin-Bestandteile, wie z.B. Ammoniak, sehr stark in der Urin-Flüssigkeit, insbesondere in dem darin enthaltenen Wasser, gebunden sind und daher nicht in den erforderlichen Konzentrationen in der Gasphase vorliegen.

- abhängig vom Alter der Urinprobe gewisse Stoffe, wie z.B. Amine, Alkohole etc. bei der Messung noch nicht oder nicht mehr vorliegen.

Da eine Erwärmung der Wandungen von Kunststoff-Flaschen bzw. Kunststoff-Behältern über ca. 60 °C aus materialtechnischen Gründen nicht möglich ist, ist die Erzeugung von für einen Urinnachweis erforderlichen Partialdrücken charakteristischer Urin-Inhaltsstoffe mit großen Unsicherheiten verknüpft.

Der Erfindung liegt die Aufgabe zugrunde, ein Verfahren und eine Vorrichtung vorzuschlagen, welche ohne Beeinträchtigung der Materialeigenschaften der Flaschen/Behälter eine sichere Urindetektion, insbesondere Spurenanalyse von Resten von Urin, ermöglichen, d.h. einerseits, daß alle mit Urin behafteten Flaschen/Behälter detektiert werden und andererseits, daß das neue Verfahren nicht zu einer unerwünschten Ausschleusung von nicht-kontaminierten Flaschen führt.

Erfindungsgemäß wird die gestellte Aufgabe bei einem gattungsgemäßen Verfahren durch die kennzeichnenden Merkmale des Anspruchs 1 und bei einer gattungsgemäßen Vorrichtung durch die kennzeichnenden Merkmale des Anspruchs 8 gelöst.

Damit wird also die nichtflüchtige Urin-Leitsubstanz Harnstoff mit hoher Empfindlichkeit, bei gleichzeitig kurzer Meßzeit, selektiv detektiert. Der Reaktions-Stoff wird vorzugsweise an der Innenoberfläche des Behälters fein verteilt; es erfolgt eine Zersetzung des Harnstoffes in u.a. Ammoniak und Kohlendioxid, und diese Folgeprodukte werden zum Nachweis des Urins herangezogen. Der Reaktions-Stoff wirkt also als Katalysator des Zersetzungsvorganges des Harnstoffs.

Dies wird gemäß der technischen Lehre der Erfindung insbesondere dadurch erreicht, daß als Reaktions-Stoff eine wässrige Lösung des Enzyms UREASE in die Flasche eingedüst und daß als Reaktionsprodukte Ammoniak und Kohlendioxid erfaßt werden und/oder daß als Reaktions-Stoff Natronlauge zugegeben, insbesondere eingedüst wird.

Wesentliche Vorteile dieser neuen Lösung liegen darin, daß nur geringe Mengen an UREASE oder NaOH im Mikroliter-Bereich benötigt werden, was eine wirtschaftliche Lösung des Problems ermöglicht. Ferner ist UREASE bzw. NaOH ein ungefährlicher Stoff, der bereits bei leicht erhöhter Zimmertemperatur in völlig schadlose Bestandteile zerfällt, die in der nachfolgenden Spülmaschine der Abfüllanlage quantitativ ausgewaschen werden. Außerdem wird durch dieses Verfahren eine sehr schnelle Urin-Detektion im Sekunden- bzw. Subsekunden-Bereich erreicht, so daß unter anderem auch bei hohen Flaschen-/Behälter-Durchsätzen, z.B. von ca. 40.000 Flaschen/Stunde, eine sichere Urinerkennung möglich ist.

Insbesondere ist auch eine sehr empfindliche Detektion im ppm- bzw. im sub-ppm-Bereich nach diesem Verfahren möglich, so daß eine sichere Urindetektion auch nach Spülung der Flasche/des Behälters mit Wasser oder anderen Lösungsmitteln und anschließend langen Standzeiten bei geöffnetem Verschluß gewährleistet ist.

In bevorzugter Ausgestaltung sieht die Erfindung einen $CO_2$-Laser vor, so daß die Detektion mittels dieses $CO_2$-Lasers in dem Arbeitsbereich von ca. 10 μm erfolgt.

Die Erfindung wird anhand der Figuren 1 bis 2 näher beschrieben. Dabei zeigen:

Figur 1      das Grundprinzip des erfindungsgemäßen Verfahrens und ein Beispiel einer erfindungsgemäßen Vorrichtung,

Figur 2      das Grundprinzip eines Gasanalysesystems zur online-Detektion der Harnstoff-Folgeprodukte.

Behälter 1 in Form von Flaschen werden über ein Transportsystem 2 zur online-Analysenstation 12,13 befördert. Ein thermostatisierter Kühlbehälter 5, der sich in einem Kühlsystem 4 befindet, wird mit einer wässrigen Lösung 3 des Enzyms UREASE bzw. NaOH über den Stutzen 17 befüllt. Über eine Dosierpumpe 7 und eine Zuleitung 6 wird mittels einer Sprühdose 8 die Innenwandung 10 der jeweiligen Flasche 1 mit der UREASE- bzw. NaOH-Lösung 3 flächendeckend benetzt. Die durch das Enzym UREASE bzw. NaOH katalysierte Reaktion

Harnstoff → Ammoniak + Kohlendioxid

$$CO\ (NH_2)_2 + H_2O \rightarrow 2NH_3 + CO_2 \qquad (1B)$$

führt zur Freisetzung der Gase Ammoniak und Kohlendioxid, die über ein Probenahmesystem 12 einem Gasanalysesystem 13 zugeführt werden, welches bei Nachweis der Gase $NH_3$/ $CO_2$ und damit von Harnstoff eine Ausschleusung der betreffenden Flasche 1' über ein Ausleitsystem 14 bewirkt.

Erfindungsgemäß ist die UREASE- bzw. NaOH-Eindüsung (8) mindestens die Strecke 11 vom Probenahmesystem 12 entfernt, so daß die enzymatische Reaktion gemäß Beziehung 18, d. h. die Bildung der Gase $NH_3$ und $CO_2$, weitgehend abgeschlossen ist.

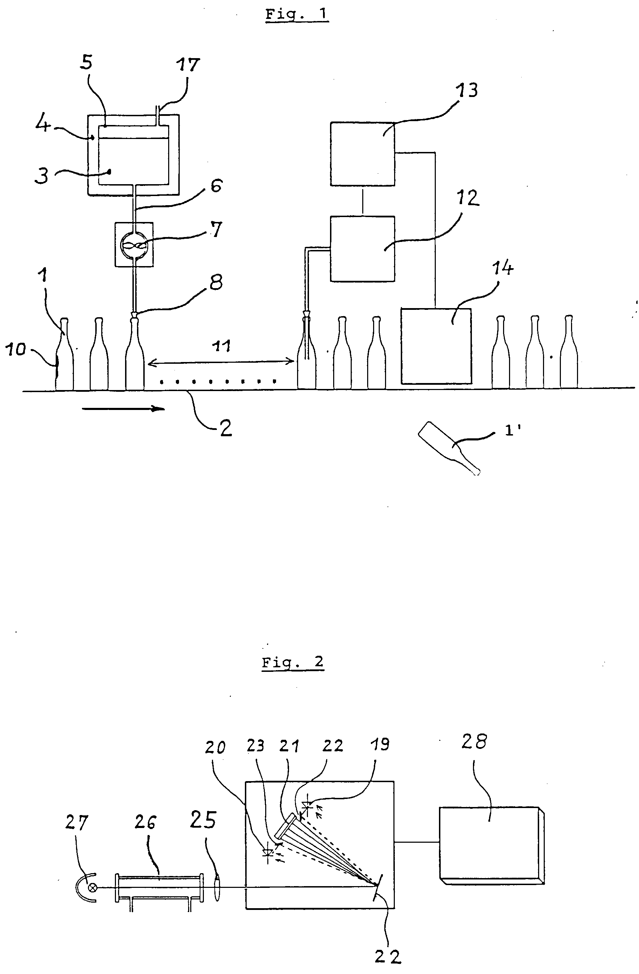

In einer bevorzugten Ausbildung der Erfindung wird das gemäß der europäischen Anmeldung EP-A-0520322 offenbarte Gasanalysesystem eingesetzt und gemäß Fig. 2 so erweitert, daß die Gase $NH_3$ und $CO_2$ durch zwei zusätzliche Infrarot-Sensoren 19,20 quantitativ gemessen werden, die so angeordnet sind, daß sie z.B. über Spiegel 22,23 ausgekoppelte Wellenlängen bei 3,0 μm, entsprechend der Absorption von $NH_3$, und 4,2 μm, entsprechend der Absorption von $CO_2$, erfassen. Alternativ können auch andere Wellenlängen, z.B. 2,6 μm für $CO_2$ und 1,5 μm oder 9,5 μm für $NH_3$ gewählt werden. In letzterem Falle kann insbesondere ein $CO_2$-Laser eingesetzt werden.

Das Sensorarray 21, das optische Gitter 22 und die übrigen optischen Komponenten 25, 26, 27 entsprechen denen der EP-A-0520322.

Um die Sicherheit der Urin-Detektion auf de facto 100 % zu bringen, werden in einer bevorzugten Ausführung des Systems die Konzentrationen beider Gase, d. h. $CO_2$ und $NH_3$, simultan ermittelt, d.h. eine Aussortierung der jeweiligen Flasche/des Behälters erfolgt nur dann, wenn sowohl eine gegenüber dem Untergrund erhöhte Konzentration von $CO_2$ als auch von $NH_3$ vorliegt und wenn die Erhöhung im durch Gleichung 18 vorgegebenen stöchiometrischen Verhältnis der Gase $CO_2$/$NH_3$ erfolgt.

Die UREASE-Lösung 3 hat in einer besonders wirtschaftlichen Ausgestaltung der Erfindung folgende Zusammensetzung:

- 1 g UREASE gelöst in
- 5 l Wasser.

In gleicher Weise kann eine niedrig-prozentige NaOH-Lösung eingesetzt werden.

Die Temperatur der Lösung 3 beträgt vorzugsweise 7 °C. Dementsprechend wird die Temperatur des Behälters 4 ebenfalls auf 7 °C eingestellt. Bezogen auf eine Polyethylene-Flasche mit einem Füllvermögen von 1,5 1 werden je 0,5 bis 5 g der obengenannten UREASE/Wasser- bzw. NaOH-Lösung mit dem System 8 eingedüst. Bei anderen Flaschengrößen/Behältervolumina werden proportional zur veränderten Innenoberfläche des Gefäßes veränderte Lösungsmengen eingespritzt.

Die Konzentrationsbestimmung der Folgeprodukte $NH_3$ und/ oder $CO_2$ erfolgt entsprechend der Lehre zum technischen Handeln gemäß der EP-A-0520322, wobei von einem Signalprozessor 28 ermittelt wird, inwieweit die $CO_2$- und/oder $NH_3$-Konzentrationen über dem jeweiligen Untergrund in den Flaschen/Behältern erhöht wird und ob das Verhältnis der Konzentrationserhöhungen dem stöchiometrischen Verhältnis gemäß Gleichung 18, innerhalb einer vorgegebenen Toleranz T, entspricht. In einem speziellen Ausführungsbeispiel kann T so gewählt werden, daß $T \leq 10\ \%$ ist.

## Patentansprüche

1. Verfahren zur Detektion und zur quantitativen Analyse von Resten von Urin in Behältern (1, 1'), wie Flaschen, insbesondere in der Getränkeindustrie, dadurch gekennzeichnet, daß in den Behälter (1, 1') ein Reaktions-Stoff (3) eingebracht wird, der durch chemische Reaktion mit mindestens einem Bestandteil des Urins zu einem spektroskopisch nachweisbaren, flüchtigen Nachweis-Stoff führt.

2. Verfahren nach Anspruch 1, dadurch gekennzeichnet, daß als Reaktions-Stoff (3) eine wässrige Lösung des Enzyms UREASE in die Flasche eingedüst und daß als Reaktionsprodukte Ammoniak und Kohlendioxid erfaßt werden.

3. Verfahren nach Anspruch 1, dadurch gekennzeichnet, daß als Reaktions-Stoff (3) Natronlauge zugeben, insbesondere eingedüst wird.

4. Verfahren nach einem der Ansprüche 1 bis 3, dadurch gekennzeichnet, daß die Eindüsung mindestens 1 Sekunde vor der Erfassung der Reaktionsprodukte erfolgt.

5. Verfahren nach einem der Ansprüche 1 bis 4, dadurch gekennzeichnet, daß sich der Reaktions-Stoff in einem thermostatisierten Kühlbehälter (5) befindet.

6. Verfahren nach einem der Ansprüche 1 bis 5, dadurch gekennzeichnet, daß eine Aussortierung eines Behälters (1') erfolgt, wenn die Konzentratio-

nen der Reaktionsprodukte $CO_2$ und/oder $NH_3$ oberhalb vorgebbaren Toleranzen über den zugrundegelegten Untergrund-Werten in dem Behälter (1') liegen.

7. Verfahren nach einem der Ansprüche 1 bis 6, dadurch gekennzeichnet, daß eine Aussortierung nur dann erfolgt, wenn die gegenüber dem Untergrund gemessenen Verhältnisse der Konzentrationen von $NH_3$ und/oder $CO_2$ sich innerhalb vorgegebener Toleranzen wie 1:2 verhalten.

8. Vorrichtung zur Detektion und zur quantitativen Analyse von Resten von Urin in Behältern (1, 1'), wie Flaschen, insbesondere in der Getränkeindustrie, gekennzeichnet durch eine Einrichtung (5-8) zum Einbringen eines Reaktions-Stoffes (3) in die Behälter (1, 1'), wobei der Reaktions-Stoff (3) durch chemische Reaktion mit mindestens einem Bestandteil des Urins zu einem spektroskopisch nachweisbare, flüchtigen Nachweis-Stoff führt.

9. Vorrichtung nach Anspruch 8, gekennzeichnet durch eine Düseneinrichtung (8) zum Eindüsen einer Lösung des Enzyms UREASE (3) in die Behälter (1, 1') und eine Erfassungseinrichtung (12) zum Erfassen der Reaktionsprodukte Ammoniak und Kohlendioxid.

10. Vorrichtung nach Anspruch 8, gekennzeichnet durch Natronlauge als Reaktions-Stoff (3).

11. Vorrichtung nach einem der Ansprüche 8 bis 10, dadurch gekennzeichnet, daß die Düseneinrichtung (8) den Reaktions-Stoff (3) mindestens 1 Sekunde vor Erfassung der Reaktionsprodukte eindüst.

12. Vorrichtung nach einem der Ansprüche 8 bis 11, gekennzeichnet durch einen thermostatisierten Kühlbehälter (5) für den Reaktions-Stoff (3).

13. Vorrichtung nach einem der Ansprüche 8 bis 12, gekennzeichnet durch eine Aussortiereinrichtung (14) zum Aussortieren eines Behälters (1'), wenn für diesen die Konzentrationen der Reaktionsprodukte $CO_2$ und/oder $NH_3$ oberhalb vorgebbaren Toleranzen über den zugrundegelegten Untergrund-Werten in dem Behälter (1') liegen.

14. Vorrichtung nach einem der Ansprüche 11 bis 13, gekennzeichnet durch eine Aussortierung nur dann, wenn die gegenüber dem Untergrund gemessenen Verhältnisse der Konzentrationen von $NH_3$ und/oder $CO_2$ sich innerhalb vorgegebener Toleranzen wie 1:2 verhalten.

**Claims**

1. Method for the detection and quantitative analysis of urine residues in containers (1, 1'), such as bottles, particularly in the beverages industry, characterized in that into the container (1, 1') is introduced a reaction substance, which leads through chemical reaction with at least one constituent of the urine to a spectroscopically detectable, volatile detection substance.

2. Method according to claim 1, characterized in that as the reaction substance (3) an aqueous solution of the enzyme UREASE is injected into the bottle and that ammonia and carbon dioxide are detected as reaction products.

3. Method according to claim 1, characterized in that caustic soda solution is added, particularly injected as the reaction substance (3).

4. Method according to one of the claims 1 to 3, characterized in that injection takes place at least one second prior to the detection of the reaction products.

5. Method according to one of the claims 1 to 4, characterized in that the reaction substance is in a thermostatted cooling container (5).

6. Method according to one of the claims 1 to 5, characterized in that there is an elimination of a container (1') if the concentrations of the reaction products $CO_2$ and/or $NH_3$ are above predeterminable tolerances over the basic background values in the container (1').

7. Method according to one of the claims 1 to 6, characterized in that elimination only takes place if the ratios of the concentrations of $NH_3$ and/or $CO_2$ measured against the background are within predetermined tolerances such as 1:2.

8. Apparatus for the detection and quantitative analysis of urine residues in containers (1, 1'), such as bottles, particularly in the beverages industry, characterized by a device (5-8) for introducing a reaction substance (3) into the container (1, 1'), the reaction substance (3) by chemical reaction with at least one constituent of the urine leading to a spectroscopically detectable, volatile detection substance.

9. Apparatus according to claim 8, characterized by an injection device (8) for injecting a solution of the enzyme UREASE (3) into the container (1, 1') and a detection device (12) for detecting the reaction products ammonia and carbon dioxide.

**10.** Apparatus according to claim 8, characterized by caustic soda solution as the reaction substance (3).

**11.** Apparatus according to one of the claims 8 to 10, characterized in that the injection device (8) injects the reaction substance (3) at least one second prior to the detection of the reaction products.

**12.** Apparatus according to one of the claims 8 to 11, characterized by a thermostatted cooling container (5) for the reaction substance (3).

**13.** Apparatus according to one of the claims 8 to 12, characterized by an eliminating device (14) for eliminating a container (1'), if for the latter the concentrations of the reaction products $CO_2$ and/or $NH_3$ are above predeterminable tolerances over the basic background values in the container (1').

**14.** Apparatus according to one of the claims 11 to 13, characterized in that an elimination only takes place if the ratios of the concentrations of $NH_3$ and/or $CO_2$ measured against the background are within predeterminable tolerances such as 1:2.

**Revendications**

**1.** Méthode pour la détection et l'analyse quantitative de résidus d'urine dans des récipients (1,1') tels que des bouteilles, en particulier dans l'industrie des boissons, caractérisée en ce que l'on introduit dans le récipient (1,1') un réactif (3) produisant par réaction chimique avec au moins une composante de l'urine un produit d'identification volatil détectable par spectroscopie.

**2.** Méthode selon la revendication 1, caractérisée en ce que l'on injecte à titre de réactif (3) une solution aqueuse d'enzyme UREASE dans la bouteille et que l'on détecte comme produits réactionnels de l'ammoniaque et du dioxyde de carbone.

**3.** Méthode selon la revendication 1, caractérisée en ce que l'on ajoute, plus particulièrement que l'on injecte à titre de réactif (3) de l'hydroxyde de sodium.

**4.** Méthode selon l'une quelconque des revendications 1 à 3, caractérisée en ce que l'injection est effectuée au moins une seconde avant la détection des produits réactionnels.

**5.** Méthode selon l'une quelconque des revendications 1 à 4, caractérisée en ce que le réactif se trouve dans une enceinte réfrigérée et thermostatée.

**6.** Méthode selon l'une quelconque des revendications 1 à 5, caractérisée en ce que l'on retire un récipient (1') lorsque les valeurs de concentrations des produits réactionnels $CO_2$ et/ou $NH_3$ dans le ré-cipient (1') dépassent les valeurs de bruit de fond prises pour référence au-delà des tolérances prédéfinies.

**7.** Méthode selon l'une quelconque des revendications 1 à 6, caractérisée en ce que l'on ne procède au retrait que lorsque les rapports des concentrations en $NH_3$ et/ou $CO_2$ mesurées par rapport au bruit de fond se situent à l'intérieur de tolérances prédéfinies telles que 1:2.

**8.** Dispositif pour la détection et l'analyse quantitative de résidus d'urine dans des récipients (1,1') tels que des bouteilles, en particulier dans l'industrie des boissons, caractérisé par une installation (5-8) pour l'introduction d'un réactif (3) dans les récipient (1,1'), réactif (3) produisant par réaction chimique avec au moins une composante de l'urine un produit d'identification volatil détectable par spectroscopie.

**9.** Dispositif selon la revendication 8, caractérisé par un appareil d'injection (8) pour l'injection d'une solution d'enzyme UREASE (3) dans les contenants (1,1') et un appareil de détection (12) pour la détection des produits réactionnels à savoir l'ammoniaque et le dioxyde de carbone.

**10.** Dispositif selon la revendication 8; caractérisé en ce que le réactif (3) utilisé est de l'hydroxyde de sodium.

**11.** Dispositif selon l'une quelconque des revendications 8 à 10, caractérisé en ce que l'appareil d'injection (8) injecte le réactif (3) au moins 1 seconde avant la détection des produits réactionnels.

**12.** Dispositif selon l'une quelconque des revendications 8 à 11, caractérisé par une enceinte (5) frigorifiée et thermostatée pour le réactif (3).

**13.** Dispositif selon l'une quelconque des revendications 8 à 12, caractérisé par un appareil de retrait (14) pour retirer un récipient (1') lorsque dans celui-ci, les concentrations des produits réactionnels $CO_2$ et/ou $NH_3$ dépassent les valeurs de bruit de fond prises pour référence au-delà de tolérances prédéfinies.

**14.** Dispositif selon l'une quelconque des revendications 11 à 13, caractérisé en ce que l'on ne procède à un retrait que lorsque les rapports des concentrations en $NH_3$ et/ou $CO_2$ mesurées par rapport au bruit de fond se situent à l'intérieur de tolérances prédéfinies telles que 1:2.

Fig. 1

Fig. 2